# EUROPÄISCHE PATENTSCHRIFT

(11) **EP 0 635 280 B1**
(45) Veröffentlichungstag und Bekanntmachung des Hinweises auf die Patenterteilung: **28.02.2001**
(21) Anmeldenummer: 94108230.7
(22) Anmeldetag: 27.05.1994
(51) Int. Cl.: A61N 1/05

(54) **Anordnung zum Formen eines Elektrodenkabels**
Device for shaping an electrode
Appareil pour la mise en forme d'une électrode

(30) Priorität: 22.07.1993 SE 9302476
(43) Veröffentlichungstag der Anmeldung: 25.01.1995
(73) Patentinhaber: St. Jude Medical AB, 175 84 Järfälla (SE)
(72) Erfinder: Nyman, Per, S-182 64 Djursholm (SE)
(74) Vertreter: Lettström, Richard Wilhelm

(56) Entgegenhaltungen:
- US-A- 3 729 008
- US-A- 3 890 977
- US-A- 4 136 703
- US-A- 4 402 328
- US-A- 4 677 990

## Beschreibung

Die Erfindung bezieht sich auf ein Elektrodenkabel für eine Herzschrittmacherelektrode und eine Anordnung zum Formen des Elektrodenkabels.

Es ist seit langem bekannt, dass bei einer Stimulierung eines Aurikels eines Patienten ein vorgebogenes J-förmiges Elektrodenkabel verwendt wird, damit der für die Stimulierung vorgesehene Elektrodenkopf, der gegen die Aurikelwand anliegen soll, abgestützt wird. In der US-PS 4 402 328 ist ein solches Elektrodenkabel beschrieben. Mit Hilfe des vorgebogenen Elektrodenkabels kommt der Elektrodenkopf im Aurikel zu liegen ohne danach zu streben, sich aus dieser Lage zu verschieben.

Eine weitere Anordnung zum Formen eines Elektrodenkabels ist durch die US-PS 4 136 703 bekannt. In den Mandrinkanal des Elektrodenkabels ist ein verhältnismässig steifes Rohr, das sich über die Länge des Elektrodenkabels erstreckt, eingeschoben. Im Rohr ist ein J-förmig vorgebogener Mandrin, der mit Hilfe des Rohres ausgerichtet ist, eingeführt. Wenn sich das distale Ende des Elektrodenkabels im Herz befindet, wird das Rohr nach hinten verschoben, so dass der Mandrin freigelegt wird, der somit das distale Ende des Kabels formt. Ein Elektrodenkabel dieser Art ist extrem steif. Bei dem Entfernen des Mandrins vom Elektrodenkabel würde ein im Aurikel applizierter Elektrodenkopf aufgrund seines Eigengewichts danach streben, sich aus seiner Lage zu entfernen.

In der US-PS 3 890 977 ist eine Kombination einer Elektrode und eines Katheters, die mit Teilen aus Gedächtnismetall versehen ist, die an bestimmten Stellen entlang dem Elektroden-Katheter-Kabel appliziert sind, beschrieben. Nachdem das Kabel im Herzen eines Patienten appliziert ist, wird das Kabel mit Hilfe der Platten aus Gedächtnismetall, die von der Körpertemperatur beeinflusst werden, geformt.

Ein gemeinsamer Nachteil dieser beschriebenen Elektrodenvorrichtungen ist, dass eine grössere Auswahl von J-förmigen Elektrodenkabeln bzw. vorgebogener Mandrinen verschiedener Grössen vorhanden sein muss, da der Abstand zwischen dem gebogenenen Abschnitt des Kabels und dem Elektrodenkopf mehr oder weniger an das Herz jedes einzelnen Patienten angepasst werden muss.

Der Erfindung liegt die Aufgabe zugrunde, eine Anordnung der eingangs genannten Art zu schaffen, die in einer extrem einfachen und billigen Weise die Anpassung einer Elektrodenvorrichtung an ein Herz eines Patienten vereinfacht.

Diese Aufgabe ist erfindungsgemäss dadurch gelöst, dass die Anordnung ein hauptsächlich rohrförmiges, im Vergleich zur Länge des Elektrodenkabels kurzes Element ist, dessen Innendurchmesser an den Aussendurchmesser des Elektrodenkabels angepasst ist, und wobei das Element derart aufgebaut ist, dass es auf das Elektrodenkabel aufschiebbar ist und wenigstens teilweise das Elektrodenkabel umschliesst, wobei das Element eine derartige Steifigkeit aufweist, dass das Elektrodenkabel innerhalb der Strecke des aufgeschobenen Elements durch das Element geformt wird. Durch dieses Element, das an einer gewünschten Stelle eines Kabels einer Elektrodenvorrichtung anbringbar ist, ist eine extrem einfache und billige Art, eine Elektrodenvorrichtung entsprechend dem Herzen eines Patienten zu formen, gegeben. Ausserdem kann der Arzt eine gerade Standardelektrode, auf die das Element nach der Erfindung aufgeschoben wird, verwenden. Dies bedeutet, dass sowohl Hersteller und Wiederverkäufer als auch Ärzte in den Krankenhäusern ein vereinfachtes Elektrodensortiment und dadurch ein geringeres Elektrodenlager besitzen können.

In einer vorteilhaften Ausbildung der Erfindung wird vorgeschlagen, dass das Element vorgebogen und derart biegbar ist, dass es und das Elektrodenkabel mit Hilfe eines Mandrins auch im Bereich dieser Strecke aufrichtbar sind. Hierdurch wird erreicht, dass die Elektrodenvorrichtung in bekannter Weise in das Herz über eine Vene einführbar ist.

In einer Weiterbildung der Erfindung wird vorgeschlagen, dass das Element U- oder J-förmig ausgebildet ist. Auf diese Weise wird, wenn das Element auf eine nicht gebogene Standardelektrode aufgeschoben wird, einfach ein J-förmiges Elektrodenkabel erhalten.

Eine weitere günstige Ausgestaltung der Erfindung ergibt sich, wenn das Element wenigstens teilweise aus einem Gedächtnismetall von einer solchen Art, dass das Element bei Körpertemperatur eine gewünschte Form einnimmt, besteht. Durch diese Wahl des Materials kann das Element auch auf einer Elektrodenvorrichtung, die ohne Hilfe eines Mandrins in das Herz einführbar ist, aufgeschoben werden. Das Element, das hier aus einem Gedächtnismetall hergestellt ist das in das Herz in einer gewünschten Form gebogen wird und auch gleichzeitig das Elektrodenkabel formt, kann bei der Einführung etwa gerade sein.

Im Hinblick auf eine günstige Ausgestaltung der Erfindung empfiehlt es sich, dass das Element derart ausgebildet ist, dass es auf dem Elektrodenkabel verschiebbar ist. Auf diese Weise kann auf eine schnelle und unkomplizierte Weise eine Lageänderung des Elements und damit eine Änderung des Abstandes zwischen dem Element und dem Elektrodenkopf am Kabel durchgeführt werden.

In einer vorteilhaften Ausführungsform der Erfindung wird vorgeschlagen, dass das Element über seine gesamte Länge mit einem Schlitz versehen ist. Hierdurch kann das Element schnell und einfach auf das Elektrodenkabel aufgebracht bzw. vom Elektrodenkabel entfernt werden.

In einer weiteren vorteilhaften Ausführungsform der Erfindung wird vorgeschlagen, dass der Schlitz an den Endbereichen des Elements wenigstens teilweise wendelförmig verläuft. Auf diese Weise sind die Enden des Elements um das Elektrodenkabel geschlossen.

Eine konstruktiv einfache Ausführung der Erfindung ist dadurch gegeben, dass das Element wendelförmig ausgebildet ist. Hierdurch wird das Element sehr leicht. Ausserdem kann das Element durch diese Form auf das Elektrodenkabel aufgeschraubt bzw. aufgedreht werden.

Weitere Vorteile und Einzelheiten ergeben sich aus den Unteransprüchen.

Die Erfindung ist nachfolgend anhand mehrerer in den Zeichnungen dargestellten Ausführungsbeispielen näher erläutert.
Es zeigen:

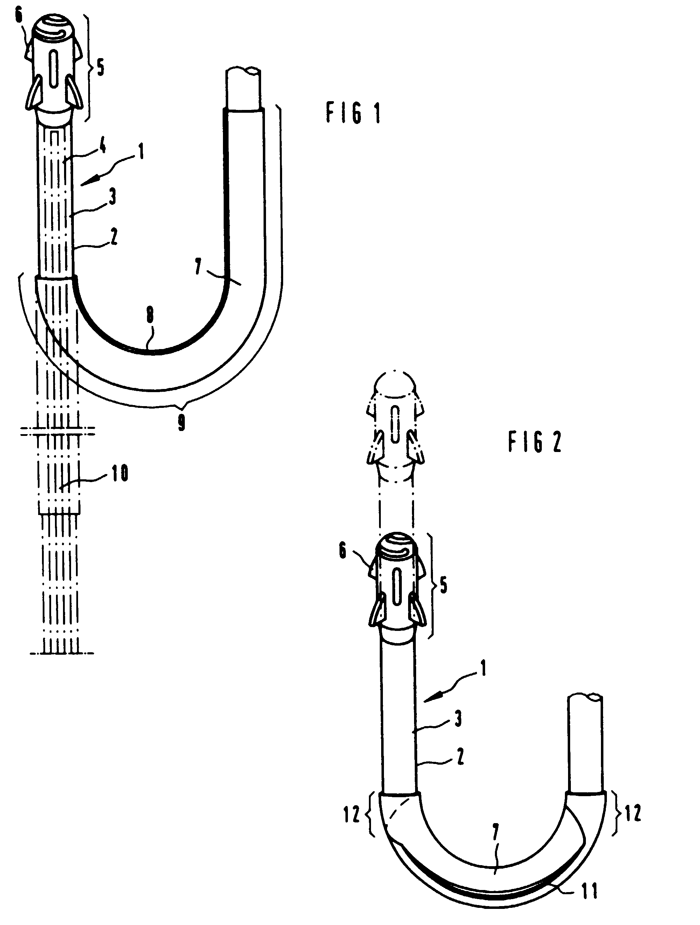

- FIG 1 bis 4: Seitenansichten eines Elements nach der Erfindung in verschiedenen Ausführungsformen, die auf einem Elektrodenkabel appliziert sind.

In der FIG 1 ist eine Herschrittmacherelektrode 1 von einer nicht vorgebogenen Standardausführung gezeigt, bestehend aus einem Elektrodenkabel 2 mit einem langgestreckten, flexiblen, wendelförmigen hier nicht gezeigten Leiter, dessen Aussenseite mit einer Isolierschicht 3 versehen ist und dessen Innenseite ein Kanal 4 bildet. Am distalen Ende des Leiters bzw. des Elektrodenkabels 2 ist ein Elektrodenkopf 5 zur Stimulierung des Herzgewebes eines Patienten angeschlossen. Am Elektrodenkopf 5 sind auch flossenförmige Fixierelemente 6, die zum Fixieren des Elektrodenkopfes 5 an der Herzwand vorgesehen sind, angeordnet. Wenn der Arzt beabsichtigt, diese Standardelektrode für die Stimulation des Aurikels im Herzen eines Patienten zu verwenden, wird das Element 7 auf das Elektrodenkabel 2 aufgeschoben.

Das Element 7, das zum Formen des Elektrodenkabels 2 vorgesehen ist, ist hauptsächlich rohrförmig und im Verhältnis zur Länge des Elektrodenkabels 2 kurz. Der Innendurchmesser des Elements 7 ist an dem Aussendurchmesser des Elektrodenkabels 2 angepasst, so dass das Element 7 auf das Elektrodenkabel 2 aufschiebbar ist. In dem in der FIG 1 gezeigten Ausführungsbeispiel ist das Element 7, das hier J-förmig ausgebildet ist, über seine gesamte Länge mit einem Schlitz 8 versehen. Auf diese Weise ist es einfach, das Element 7 auf das Elektrodenkabel 2 zu applizieren. Das Element 7 weist eine solche Steifigkeit auf, dass das Elektrodenkabel 2 innerhalb der Strecke 9 des aufgeschobenen Elements 7 durch das Element 7 geformt wird. Da das Element 7 vorzugsweise so ausgebildet ist, dass es auf das Elektrodenkabel 2 verschiebbar ist, kann der Arzt nunmehr nach Bedarf den Abstand zwischen dem J-förmigen Element 7 und dem Elektrodenkopf 5 ändern. Das Element 7 kann, um die richtige Länge des erwähnten Abstandes am Elektrodenkabel 2 zu erhalten, auch vom Elektrodenkabel 2 entfernt und anderswo wieder auf das Elektrodenkabel 2 aufgeschoben werden. Das Element 7 ist auch derart biegbar, dass dieses und das Elektrodenkabel 2 mit Hilfe eines Mandrins aufrichtbar ist. Dies ist mittels der punktgestrichelten Konturen des Elements 7 und des Elektrodenkabels 2 in der FIG 1 dargestellt. Hierdurch kann das Elektrodenkabel 2 wie üblich über eine Vene in das Herz eingeführt werden.

In der FIG 2 ist eine weitere Ausführungsform des Elements 7 gezeigt, das hier mit einem längsverlaufenden Schlitz 11 versehen ist, der an den Endbereichen 12 des Elements 7 wendelförmig gedreht ist. Diese Wendelform des Schlitzes 11 soll als Verschluss für die Enden des Elements 7 dienen. In dieser FIG ist mit Hilfe der punktgestrichelten Version des distalen Endes des Elektrodenkabels 2 gezeigt, dass der Abstand zwischen dem Elektrodenkopf 5 und dem Element 7, wie bereits beschrieben, geändert werden kann.

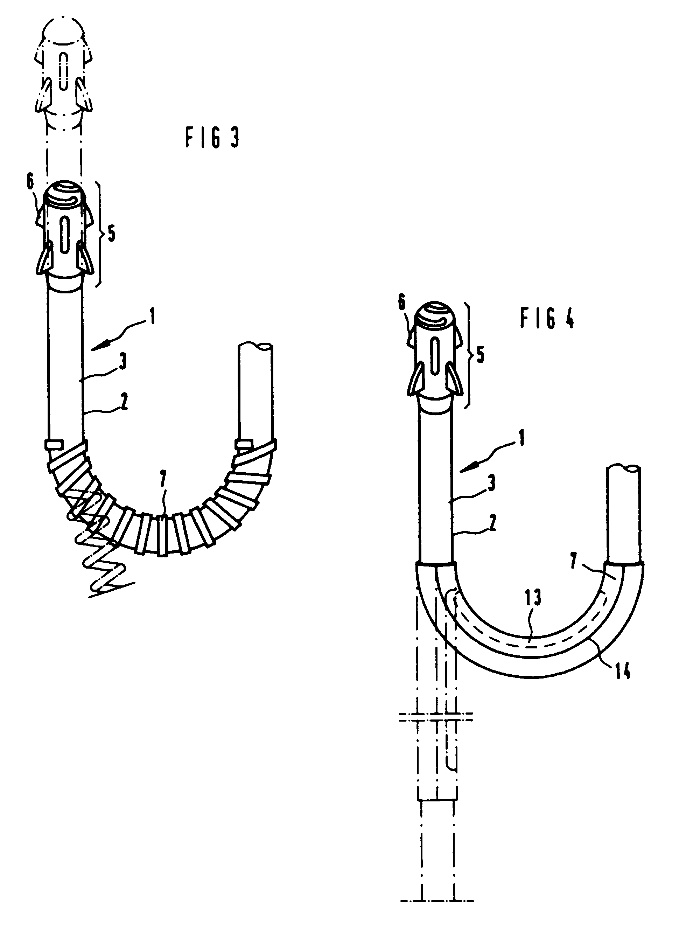

In der FIG 3 ist dargestellt, dass das Element 7, das hier U-förmig ausgebildet ist, als eine mehr oder weniger dicht gewickelte Wendel ausgebildet werden kann. Das Element 7 kann in dieser Ausführungsform, wie es in der FIG. mit den punktgestrichtelten Konturen dargestellt ist, entweder seitlich auf das Elektrodenkabel 2 aufgeschraubt oder auf das Elektrodenkabel 2, z.B. von dessen proximalen Ende, aufgeschoben werden. Auch dieses Element 7 kann, wie es in der FIG mit Hilfe des punktgestrichelten distalen Endes des Elektrodenkabels gezeigt ist, auf dem Elektrodenkabel 2 verschoben werden, so dass der Abstand zwischen dem Element 7 und dem Elektrodenkopf 5 geändert werden kann. Das Element 7 in diesem Ausführungsbeispiel kann aus einem derartigen Gedächtnismetall bestehen, dass das Element 7 bei Körpertemperatur eine gewünschte Form, d.h. in diesem Falle eine U-Form einnimmt. Dies bedeutet, dass der Arzt eine solche Elektrodenvorrichtung verwenden kann, die ohne Hilfe eines Mandrins über eine Vene in das Herz einführbar ist, da das Element 7 bei der Einführung etwa gerade sein kann und in beschriebener Weise im Herzen automatisch die gewünschte U-form einnimmt.

In der FIG 4 ist eine weitere Ausführungsform eines Elements 7 gezeigt, die mit einer Platte 13 aus einem Gedächtnismetall versehen ist, die, wie in Verbindung mit der FIG 3 beschrieben ist, von einer etwa geraden Lage in eine vorbestimmte U-Form gebogen werden kann. Die FIG 4 soll diese Lageveränderung veranschaulichen. Das Element 7 ist ferner mit einem Schlitz 14 versehen, so dass es auf das Elektrodenkabel 2 aufgeschoben oder vom Elektrodenkabel 2 entfernt werden kann.

Das U- bzw. J-förmige Element 7 kann vorzugsweise aus einem Silikonmaterial bestehen. Das Element 7 kann auch aus einem resorbierbaren Material bestehen, so dass es lediglich den Elektrodenkopf 5 nach der Implantation abstützt, bis dieser an der Herzwand festgewachsen ist. Mit Hilfe des erwähnten Materials kann das Elektrodenkabel 2 danach seine natürliche Flexibilität im Bereich der U- oder der J-Form, was eventuell die Lebensdauer des Elektrodenkabels bzw. des Leiters erhöht, erhalten.

Durch die Erfindung kann eine nicht vorgebogene Standardelektrode mit einem aufschiebbaren Element 7 versehen werden, die rasch diese Standardelektrode in eine J-förmige Elektrode, die z.B. für die Stimulierung eines Aurikels vorgesehen ist, umwandelt. Ausserdem kann der Abstand zwischen dem U- bzw. J-förmigen Element 7 und dem Elektrodenkopf 5 schnell geändert werden, was bedeutet, dass das Elektrodenkabel 2 in einer einfachen Weise an jedem einzelnen Herz eines Patienten angepasst werden kann. Dies bedeutet ferner, wie bereits beschrieben, dass eine vereinfachte Auswahl von Elektrodenvorrichtungen und dadurch kleinerer Lager sowohl beim Hersteller und Wiederverkäufer als auch bei den Ärzten in den Krankenhäusern erhalten wird.

### Bezugszeichenliste

- 1: Herzschrittmacherelektrode
- 2: Elektrodenkabel
- 3: Isolierschicht
- 4: Kanal
- 5: Elektrodenkopf
- 6: Fixiermittel
- 7: Anordnung, Element
- 8, 11, 14: Schlitz
- 9: Strecke
- 10: Mandrin, Steuermittel
- 12: Endbereich
- 13: Platte

## Patentansprüche

1. Elektrodenkabel für eine Herzschrittmacherelektrode und eine Anordnung zum Formen des Elektrodenkabels, welche Anordnung (7) ein hauptsächlich rohrförmiges, im Vergleich zur Länge des Elektrodenkabels (2) kurzes Element (7), ist dessen Innendurchmesser an den Aussendurchmesser des Elektrodenkabels (2) angepasst ist, dadurch **gekennzeichnet**, dass das Element (7) derart aufgebaut ist, dass es auf das Elektrodenkabel (2) aufschiebbar ist und wenigstens teilweise das Elektrodenkabel (2) umschliesst, wobei das Element (7) eine derartige Steifigkeit aufweist, dass das Elektrodenkabel (2) innerhalb der Strecke des aufgeschobenen Elements (7) durch das Element (7) geformt wird.

2. Anordnung nach Anspruch 1, **dadurch gekennzeichnet**, dass das Element (7) vorgebogen und derart biegbar ist, dass es und das Elektrodenkabel (2) mit Hilfe eines Mandrins (10) auch im Bereich dieser Strecke aufrichtbar sind.

3. Anordnung nach Anspruch 1 oder 2, **dadurch gekennzeichnet**, dass das Element U- oder J-förmig ausgebildet ist.

4. Anordnung nach Anspruch 1, **dadurch gekennzeichnet**, dass das Element (7) wenigstens teilweise aus einem Gedächtnismetall von einer solchen Art, das das Element (7) bei Körpertemperatur eine gewünschte Form einnimmt, besteht.

5. Anordnung nach einem der Ansprüche 1 bis 4, **dadurch gekennzeichnet**, dass das Element (7) derart ausgebildet ist, dass es auf dem Elektrodenkabel (2) verschiebbar ist.

6. Anordnung nach einem der Ansprüche 1 bis 5, **dadurch gekennzeichnet**, dass das Element (7) über seine gesamte Länge mit einem Schlitz (8, 11, 14) versehen ist.

7. Anordnung nach Anspruch 6, **dadurch gekennzeichnet**, dass der Schlitz (11) an den Endbereichen (12) des Elements (7) wenigstens teilweise wendelförmig verläuft.

8. Anordnung nach einem der Ansprüche 1 bis 5, **dadurch gekennzeichnet**, dass das Element (7) wendelförmig ausgebildet ist.

9. Anordnung nach einem der Ansprüche 1 bis 8, **dadurch gekennzeichnet**, dass das Element (7) aus einem Silikonmaterial besteht.

10. Anordnung nach einem der Ansprüche 1 bis 8, **dadurch gekennzeichnet**, dass das Element (7) aus einem resorbierbaren Material besteht.

## Claims

1. Electrode cable for a heart pacemaker electrode and an arrangement for shaping the electrode cable, which arrangement (7) is a mainly tubular element (7), which is short in comparison with the length of the electrode cable (2) and the inside diameter of which is matched to the outside diameter of the electrode cable (2), characterised in that the element (7) is constructed in such a way that it can be slid on to the electrode cable (2) and surrounds the electrode cable (2) at least partially, the element (7) having a rigidity which is such that the electrode cable (2) inside the section of the element (7) which has been slid on is shaped by the element (7).

2. Arrangement according to claim 1,
characterised in that the element (7) is pre-bent and is flexible in such a way that it and the electrode cable (2) can be straightened out also in the region of this section with the aid of a mandrin (10).

3. Arrangement according to claim 1 or 2,
characterised in that the element is constructed such that it is U-shaped or J-shaped.

4. Arrangement according to claim 1,
characterised in that the element (7) consists at least partially of a memory-effect metal of such a type that the element (7) takes on a desired shape at body temperature.

5. Arrangement according to one of claims 1 to 4, characterised in that the element (7) is constructed in such a way that it can be shifted on the electrode cable (2).

6. Arrangement according to one of claims 1 to 5, characterised in that the element (7) is provided with a slit (8, 11, 14) over its entire length.

7. Arrangement according to claim 6,
characterised in that the slit (11) extends at least partially spirally at the end regions (12) of the element (7) .

8. Arrangement according to one of claims 1 to 5, characterised in that the element (7) is constructed in a manner such that it is spiral-shaped.

9. Arrangement according to one of claims 1 to 8, characterised in that the element (7) consists of a silicon material.

10. Arrangement according to one of claims 1 to 8, characterised in that the element (7) consists of a resorbable material.

## Revendications

1. Câble d'électrode pour une électrode de stimulateur cardiaque et un dispositif pour la mise en forme du câble d'électrode, ce dispositif (7) étant un élément (7) principalement tubulaire qui est court par rapport à la longueur du câble (2) d'électrode, et dont le diamètre intérieur est adapté au diamètre extérieur du câble (2) d'électrode, caractérisé en ce que l'élément (7) est constitué de façon à pouvoir être enfilé sur le câble (2) d'électrode et à entourer au moins partiellement le câble (2) d'électrode, l'élément (7) ayant une raideur telle que le câble (2) d'électrode est mis en forme par l'élément (7) à l'intérieur de l'étendue de l'élément (7) enfilé.

2. Dispositif suivant la revendication 1, caractérisé en ce que l'élément (7) est précourbé et est flexible de façon à ce que l'élément et le câble (2) d'électrode, puissent être redressés également dans la région de cette étendue à l'aide d'un mandrin (10).

3. Dispositif suivant la revendication 1 ou 2, caractérisé en ce que l'élément est constitué en forme de U ou de J.

4. Dispositif suivant la revendication 1, caractérisé en ce que l'élément (7) est constitué au moins en partie d'un métal à mémoire d'un type tel, que l'élément (7) prend une forme souhaitée à la température du corps.

5. Dispositif suivant l'une des revendications 1 à 4, caractérisé en ce que l'élément (7) est constitué de manière à pouvoir coulisser sur le câble (2) d'électrode.

6. Dispositif suivant l'une des revendications 1 à 5, caractérisé en ce que l'élément (7) est muni sur toute sa longueur d'une fente (8, 11, 14).

7. Dispositif suivant la revendication 6, caractérisé en ce que la fente (11) s'étend au moins en partie hélicoïdalement sur les régions (12) d'extrémité de l'élément (7).

8. Dispositif suivant l'une des revendications 1 à 5, caractérisé en ce que l'élément (7) est hélicoïdal.

9. Dispositif suivant l'une des revendications 1 à 8, caractérisé en ce que l'élément (7) est en silicone.

10. Dispositif suivant l'une des revendications 1 à 8, caractérisé en ce que l'élément (7) est en une matière résorbable.
